# EUROPÄISCHE PATENTANMELDUNG

(11) **EP 0 853 928 A1**
(43) Veröffentlichungstag der Anmeldung: **22.07.1998**
(21) Anmeldenummer: 97121913.4
(22) Anmeldetag: 12.12.1997
(51) Int. Cl.: A61F 2/30, A61F 2/34, A61F 2/36

(54) **Mehrteiliges Implantat mit Klemmring**

(30) Priorität: 23.12.1996 DE 19654106
(71) Anmelder: MAN Ceramics GmbH, 94453 Deggendorf (DE)
(72) Erfinder: Ebner, Harald, 94469 Deggendorf (DE); Weber, Siegfried, 94526 Metten (DE)
(74) Vertreter: Bogsch, Adam, Dipl.-Ing.

(57) **Zusammenfassung**

Implantat, bestehend aus mindestens zwei, mittels eines Verbindungsringes (15-19) aus Kunststoff lösbar miteinander verbindbaren Komponenten (11,12;40,41) in welchem als Verbindungsring ein Klemmring (15-19) vorgesehen ist, mit dem eine vorwiegend kraftschlüssige Verbindung der Implantat-Komponenten (11,12;40,41) bewirkbar ist.

## Beschreibung

Die Erfindung bezieht sich auf ein Implantat, bestehend aus mindestens zwei mittels eines Verbindungsringes aus Kunststoff lösbar miteinander verbindbaren Komponenten.

Dem Verschleiß ausgesetzte Implantate werden häufig aus zwei trennbar zusammengesetzten Teilen hergestellt, wobei das eine Teil zum Anwachsen mit dem Knochen des menschlichen Organismus bestimmt ist, während das andere Teil bei Bedarf vom ersten Teil getrennt und ausgewechselt werden kann. Beispiele hierzu sind Pfannen und Schäfte von Gelenkimplantaten, Zahnimplantate und dergleichen.

Zur Gewährleistung der Funktionssicherheit im implantierten Zustand ist eine zuverlässige Verbindung der Komponenten erforderlich. Wesentlich ist jedoch auch, daß die Komponenten für den Chirurgen leicht zusammensetzbar sind und andererseits problemlos voneinander wieder getrennt werden können, um den Austausch der einen Komponente zu ermöglichen.

Um diesen Anforderungen zu genügen wurden insbesondere in Zusammenhang mit Gelenkpfannen zahlreiche Ausführungen von Verbindungsringen und Formgebungen der angrenzenden Bereiche der Komponenten vorgeschlagen. Es hat sich jedoch als schwierig erwiesen, eine zuverlässige Verbindung herzustellen, die gleichzeitig leicht wieder zu lösen ist, und zwar ohne hohe Kräfte anwenden zu müssen, um eine Lockerung der im Knochen eingewachsenen Komponente zu verhindern.

In der EP 298 234 ist beispielsweise ein als Gelenkpfanne dienendes Implantat der eingangs genannten Art beschrieben, das aus einer Außenschale und einem Pfannenkörper aus unterschiedlichen Materialien besteht. Eine feste aber lösbare Verbindung beider Teile bildet ein offener Kunststoffring, der eine ringförmige Nase hat, die in einer Nut des Pfannenkörpers gelagert ist. Durch Zusammendrücken des offenen Ringes gelangt dieser bei der Montage in die Außenschale, wo der Ring gegen die Innenwand der Außenschale drückt und wobei die Ringnase in eine Vertiefung einrastet. Damit ist eine sichere Verbindung in Polrichtung gegeben. Dieser bekannte Ring gewährleistet jedoch keine spielfreie Verbindung bei unter Toleranzen schwankenden Durchmessern der Komponenten. Außerdem ist zum Lösen der einen Komponente von der im Knochenmaterial angewachsenen Komponente ein Spezialwerkzeug erforderlich, mit dem der Ring zunächst zerstört werden muß.

Andere Ausführungen, wie sie beispielsweise in US 5,049,158 gezeigt sind, bestehen in Vielkantvarianten, bei denen entweder der Verbindungsring oder eine Ringnut einer der Implantatkomponenten poligonartig ausgebildet ist. Es hat sich jedoch gezeigt, daß bei diesen Schnappverbindungen nur sehr enge Toleranzen erlaubt sind, um eine möglichst spielfreie und sichere Verbindung zu gewährleisten. Außerdem sind die Komponenten schwer einzuschnappen und nur unter Anwendung von hohen Kräften wieder voneinander zu trennen.

Der Erfindung liegt die Aufgabe zugrunde, ein Implantat der eingangs genannten Art zu entwickeln, dessen Komponenten unter Beibehaltung einer sicheren Verbindung leicht zusammengesetzt und wieder voneinander getrennt werden können.

Die Aufgabe wird erfindungsgemäß mit den Merkmalen des Anspruches 1 gelöst.

Durch die Verwendung einer vorwiegend kraftschlüssigen Verbindung, bei der der Klemmring eine radiale Kraft auf die angren zenden Wandungen der miteinander verbundenen Komponenten ausübt, werden Fertigungstoleranzen ausgeglichen, so daß sich eine spielfreie Verbindung ergibt, die außerdem gegen Rotation der Komponenten gesichert ist. Eine derartige Lösung ist bei allen Mehrkomponenten-Implantaten mit lösbarer Verbindung einsetzbar.

Die für den jeweiligen Bedarf erforderlichen Steifigkeitseigenschaften des Klemmringes können in fertigungstechnisch einfacher Art durch Veränderung von Materialvolumen ggf. bei gleichbleibender Außenkontur des Klemmringes eingestellt werden.

Gemäß einer Ausgestaltung der Erfindung besteht der Klemmring aus einem geschlossenen Kunststoffring mit inhomogener Struktur, die ein inhomogenes elastisches Verhalten hervorruft. Damit lassen sich die vorerwähnten Anforderungen optimal erfüllen. Die Inhomogenität führt dazu, daß die steiferen Bereiche für eine sichere und zusammen mit den weicheren Bereichen für die spielfreie Verbindung sorgen. Das Material der steiferen Bereiche wird beim Überwinden von engen Stellen beim Zusammensetzen oder Trennen der beiden Teile in die weicheren Bereiche verdrängt und erleichtert damit das Zusammensetzen und Trennen der beiden Komponenten.

Die Inhomogenität kann durch unterschiedliche Maßnahmen verwirklicht werden. Eine Möglichkeit ist mit gezielt angeordneten Hohlräume, die als geschlossene Hohlräume, Sackbohrungen oder durchgehende Bohrungen ausgebildet werden.

Es hat sich überraschend gezeigt, daß mit derartig ausgebildeten Klemmring trotz Verwendung eines Kunststoffes relativ hoher Steifigkeit, wie z.B.PE, die Montage bzw. Demontage der Komponenten manuell ohne Kraftübertragunsmittel zu bewerkstelligen ist.

Die Anzahl der Bohrungen sowie deren Orientierung kann unterschiedlich, ggf. in Abhängigkeit des für den Klemmring gewählten Werkstoffes ausgelegt werden. Eine Unterscheidung der Konfigurationen, Durchmesser, Tiefen oder Orientierungen der Bohrungen innerhalb desselben Ringes ist auch möglich.

Gemäß einer bevorzugten Auslegung sind durchgehende Axialbohrungen oder Sackbohrungen vorgesehen, die in Umfangsrichtung des Klemmringes regelmäßig verteilt sind. Diese Ausgestaltung ist fertigungstechnisch sehr einfach herzustellen und für die Serienfertigung geeignet.

Bei Sackbohrungen können diese bei Bedarf auch im Wechsel auf der einen und der anderen Seite des Klemminges eingebracht werden. Vorteilhaft ist es, wenn nur auf einer Seite des Klemmringes axiale Sackbohrungen vorgesehen werden. Ein derartiger Klemmring ermöglicht in Verbindung mit einer Ringförmigen Erhebung der einen Implantat-Komponente eine ausgezeichnete spielfreie Verbindung, bei der die ringförmige Erhebung in den Bereich geringeren Materialvolumens eindrückt und der massive Ringbereich als Ringwulst einer Luxation entgegen der Montagerichtung entgegenwirken.

Anstelle von Bohrungen sind auch gezielte Hohlräume möglich, die so angeordnet sind, daß in Umfansgsrichtung zwischen den Hohlräumen massive Bereiche verbleiben. Ein zentraler Hohlraum entlang des gesamten Klemmring-Umfanges ist ebenfalls möglich. Derartige Klemmringe können mit nicht durchgehenden Nuten bzw. Erhebungen der Komponenten zusammenwirken.

Gemäß einer weiteren Ausgestaltung der Erfindung ist der Klemmring aus Verbundmaterial hergestellt derart, daß der Klemmring durchgehend massiv jedoch entlang des Ringes unterschiedlich steif ist. Dieses kann durch einen Verbund mit unterschiedlichen, sich örtlich abwechselnden Materialien geschehen. Einfacher ist es, wenn der Klemmring aus einem Material hergestellt und z.B. mit Stiften eines anderen Materials versetzt wird.

Es sind auch Kombinationen von verschiedenen Ausgestaltungen in einem Klemmring möglich.

Die Erfindung ist überall dort anwendbar, wo Implantatkomponente nur temporär im Körper zusammen bleiben und wo aus physikalischen Gründen Teile unterschiedlicher Materialien zusammengesetzt werden, wie zum Beispiel bei den Pfannen von Gelenkimplantaten.

Die Erfindung wird an Hand von in der Zeichnung schematisch dargestellten Ausführungsbeispielen näher beschrieben. Es zeigen:
Fig.1 ein erstes Ausführungsbeispiel an einer Pfanne eines Hüftgelenkimplantats,
Figuren 2,3 und 4 je ein Detail aus Fig.1 und
Figuren 5 bis 8 je ein weiteres Ausführungsbeispiel.

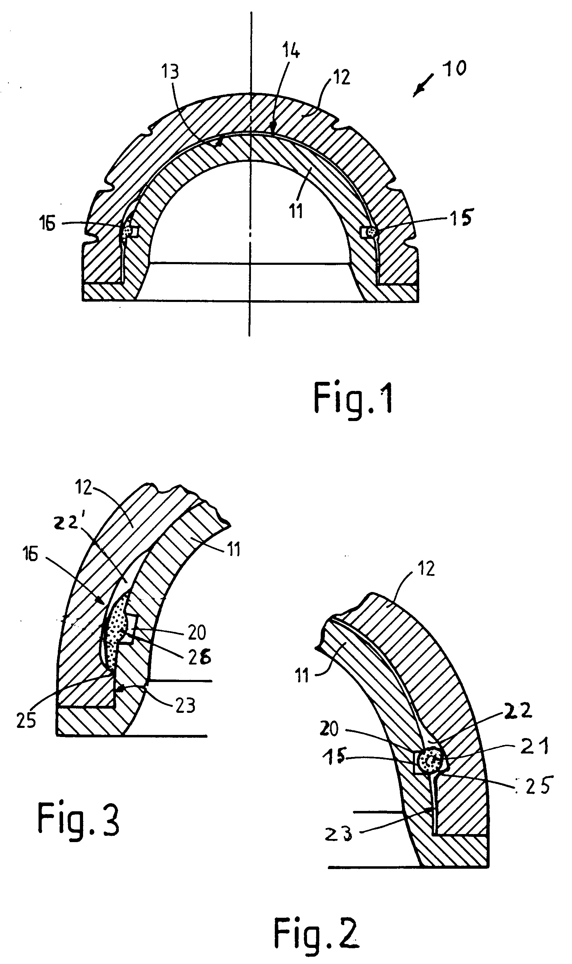

Die in Fig.1 gezeigte Pfanne 10 besteht aus einer Innenschale 11, die passgenau in eine Außenschale 12 einsetzbar ist. In der zusammengesetzten Position, wie sie in Fig.1 gezeigt ist, werden die beiden Schalen 11,12 mittels eines Klemmringes 15,(16) aus Kunststoff vorwiegend kraftschlüssig zusammengehalten. Der Klemmring wird verformt und übt dabei eine Druckkraft auf die Schalen 11,12 aus, so daß die Schalen trotz Fertigungstoleranzen sicher, spielfrei und rotationssicher verbunden sind.

Die Figuren 2 bis 4 zeigen je einen Ausschnitt von unterschiedlich ausgeführten Klemmstellen. Gemäß Fig.2 ist ein mit einer Längsbohrung 21 versehener Klemmring 15 in einer Nut 20 am Außenumfang der Innenschale 11 angeordnet, aus der er heraus ragt. Die Außenschale 12 hat an der Innenseite ebenfalls eine Nut 22 mit einer Nutkante 25. Beim Zusammensetzen der beiden Schalen 11,12 muß lediglich eine Kraft aufgewendet werden, bis die Nutkante 25 den Klemmring 15 diesen zusammendrückend überwindet. Der Klemmring 15 liegt mit Vorspannung in dem von den beiden Nuten 20,22 gebildeten Ringraum und sorgt damit für eine drehsichere, spielfrei, kraftschlüssige Verbindung der beiden Schalen 11,12. Die Hinterschneidung durch die Nutkante 25 trägt formschlüssig zur Sicherung der Verbindung gegen Trennung d.h. gegen Luxation der Innenschale 11 von der Außenschale 12 bei.

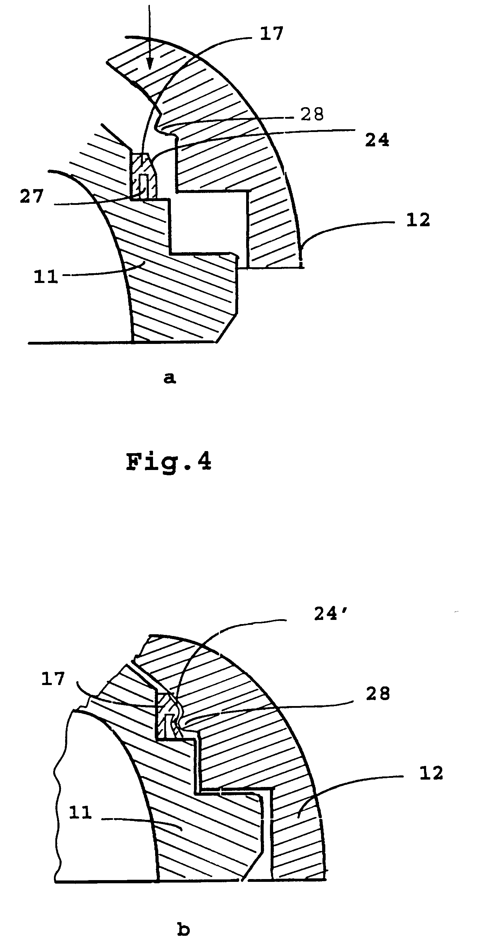

Der Querschnitt des Klemmringes kann jede Konfiguration einnehmen, d.h. rund sein oder andere Form haben. Fig.3 und 4 zeigen weitere Beispiele. Gemäß Fig.3 ist der Klemmring 16 annähernd zylindrisch mit einem nach innen gerichteten Ringwulst 26 ausgebildet. Die Ringwulst 26 ragt in die Ringnut 20 der Innenschale 11 hinein, wodurch der Klemmring 16 für die Montage oder Demontage festgehalten wird. Im zusammengesetzten Zustand übt der Klemmring 16 Radialkräfte gegen die Wandung der Nut 22' der Außenschale 12 aus.

In den Fig.4 a und b ist eine Verbindung dargestellt, die sich aus einem im Querschnitt rechteckigen Klemmring 17 mit axialen Sackbohrungen 27 und einer ringförmigen Erhebung 28 an der Innenseite der Außenschale 12 zusammensetzt. Fig.4a zeigt das Implantat 1o vor der Montage. Eine Abschrägung 24 am Klemmring 17 erleichtert die Montage, indem die Erhebung 28 den vollen Bereich des Klemmringes 17 besser überwinden kann, um dann den weicheren Bereich mit den Sackbohrungen 27 zu erreichen und zusammenzudrücken. Fig.4b zeigt das Implantat im zusammengesetzten Zustand, bei dem die Erhebung 28 in den Bereich des Klemmringes 17 mit den Sackbohrungen 27 hineindrückt. Dabei bildet der volle Bereich eine Ringwulst 24', die die Innenschale 11 gegen Luxation aus der Außenschale 12 sichert.

Beim Trennen der beiden Schalen 11,12 streifen die Nutkante 25 und die Innenwandung 23 bzw. die Erhebung 28 der Außenschale 12. über den Klemmring 15,16 bzw. 17 unter Zusammendrücken desselben. Die steiferen bzw. vollen Materialbereiche weichen dabei in die weicheren Bereiche bzw. Hohlräume aus und erlauben damit eine manuelle Trennung so wie ein manuelles Zusammenfügen der Komponenten ohne Zuhilfenahme einer Kraftübersetzung.

Herkömmliche Verbindungen wirken ausschließlich formschlüssig, so daß eine hohe Preßkraft für die Montage und eine meist noch höhere Zugkraft zum Überwinden des in der Regel relativ steifen Ring erforderlich ist. Das Lösen der Innenschale im implantierten Zustand kann daher zur Lockerung der im Knochen angewachsenen Außenschale führen. Außerdem ist es manchmal notwendig, daß vor der Implantierung das Implantat zusammengesetzt und wieder getrennt werden muß. Dieses sollte für den Chirurgen ohne Anwendung von Instrumenten möglich sein.

Bei der erfindungsgemäßen Ausgestaltung wird dagegen eine vorwiegend kraftschlüssige Verbindung hergestellt, indem ein Klemmring 15,16 oder 17 Verwendung findet, der über sein Ringvolumen inhomogen, d.h. mit unterschiedlichen Steifigkeiten ausgebildet ist. Dieses kann verschiedenartig realisiert werden.

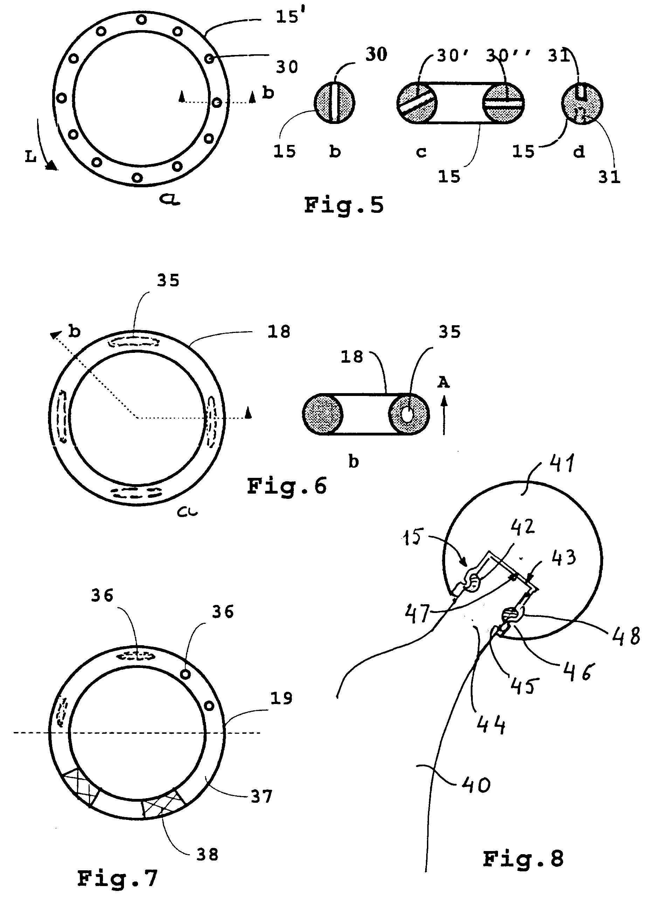

Gemäß den Ausführungsbeispielen nach Figuren 2 und 4 sind die Klemmringe 15 bzw. 17 durch eingebrachte Hohlräume 21 bzw. 27 mit örtlich verändertem Materialvolumen ausgebildet. Weitere Beispiele sind in Figuren 5 bis 7 gezeigt.

Gemäß Fig. 5 ist der Klemmring 15' mit einer Serie von axialen Bohrungen 30 versehen, die in regelmäßigen Abständen am Ring verteilt sind. Damit wechseln sich steifere Bereiche mit weni ger steifen Bereichen ab, die eine Verformung des Klemmringes während des Füge- und Trennvorganges zulassen und damit den Füge- oder Trennvorgang erleichtern und ohne Instrumentarien ermöglichen. Die Verbindung der Implantatkomponente wird durch die Vorspannung des Klemmringes im Gebrauch gesichert. Die Kombination zwischen steifen und weicheren Bereichen gewährleistet außerdem eine Anpassung des Klemmringes an die Geometrie der den Ring umgebenden Nuten, so daß Fertigungstoleranzen besser ausgeglichen werden können und dadurch spielfreie Verbindungen von Implantatteilen möglich sind.

Die Bohrungen 30 werden in der Regel den Ringquerschnitt durchdringen, wie es in den Fig.5 a,b,c gezeigt ist. Anstelle von axialen Bohrungen 30 sind auch Schrägbohrungen 30' oder Querbohrungen 30'' möglich. Insbesondere bei Klemmringen mit großem Querschnitt können Sackbohrungen 31 vorgesehen werden, die zu einer Hälfte in die eine Seite und zur anderen Hälfte versetzt in die andere Seite des Klemmringes 15 eingebracht sind, wie es in Fig.5,d gezeigt ist.

In Fig.6 a,b ist ein Klemmring 18 mit mehreren geschlossenen, auf den Umfang verteilten Hohlräumen 35 gezeigt.

Abwechselnde steifere und weniger steife Bereiche lassen sich auch mit Verbundmaterialien herstellen. In Fig. 7 sind an einem Klemmring 19 zwei Ausführungsbeispiele dargestellt. In der einen Hälfte ist ein Beispiel gezeigt, bei dem zur Versteifung in Abständen Stifte oder Einlagen 36 aus einem steiferen Material, z.B. Metall eingeschlossen sind. Gemäß der zweiten Hälfte besteht der Klemmring 19 aus zwei Materialien 37,38 unterschiedlicher Steifigkeiten, die sich entlang des Ringes abwechseln.

Die Inhomogenität wird je nach Anwendung und Bedarf gewählt. Wie in den beschriebenen Beispielen gezeigt wurde, können die mechanischen Eigenschaften sowohl in Umfangsrichtung als auch in axialer Richtung variieren.

Die Beispiele, die an Klemmringen mit rundem Querschnitt gezeigt wurden, sind auch bei Ringen mit anderen Querschnitten anwendbar. Ferner ist die Anwendung der erfindungsgemäßen Verbindung nicht auf Gelenkpfannen beschränkt.

Die Ausgestaltung und Anordnung von Nuten und Erhebungen an den Implantatkomponenten wird zur Optimierung der lösbaren Verbindung herangezogen.
Bei den Beispielen gemäß Figuren 2 und 3 ist die Verbindung durch Hinterschneidung des größten Durchmessers des Klemmringes 15 bzw. 16 der Nutkante 25 der Außenschale gesichert. Für die spielfreie Verbindung und die Rotationssicherheit sorgt die Vorspannkraft des Klemmringes, wobei die Nutkante 25 ebenfalls dazu beitragen kann, indem sie nach dem Überwinden des größten Ringdurchmessers in den Klemmring hineingedrückt bleibt. Im Fall des in Fig. 4 gezeigten Beispieles wird die Vorspannung durch die Zusammenwirkung des Klemmringes 17 mit der in den Ring hineindrückenden Erhebung 28 erreicht. Der Klemmring befindet sich hierbei nicht in einer Nut der Innenschale 11.

In Fig.8 sind als Komponenten einer Klemmverbindung ein Schaft 40 und eine Gelenkkugel 41 eines Gelenkimplantats gezeigt. In einer Ringnut 42 am Schaftstutzen 44 ist der Klemmring 15 mit rundem Querschnitt angeordnet. Eine an der Innenfläche 45 der Gelenkkugel 41 vorgesehene Ringnase 46 ist so angeordnet, daß sie nach Überwindung des größten Durchmessers des Klemmringes 15 noch eine Druckkraft auf den Klemmring auszuüben vermag, wenn die Stirnseite 43 des Schaftstutzens 44 mit der inneren Stirnseite 47 der Gelenkkugel 41 auf Anschlag steht. In dieser Stellung entlastet sich der Klemmring 15 teilweise unter Bildung einer Ringwulst oberhalb der Ringnase 46 und dringt in eine Ringnut 48 ein.

## Patentansprüche

1. Implantat, bestehend aus mindestens zwei, mittels eines Verbindungsringes aus Kunststoff lösbar miteinander verbindbaren Komponenten, dadurch gekennzeichnet, daß als Verbindungsring ein Klemmring (15-19) vorgesehen ist, mit dem eine vorwiegend kraftschlüssige Verbindung der Implantat-Komponenten (11, 12;40,41) bewirkbar ist.

2. Implantat nach Anspruch 1, dadurch gekennzeichnet, daß der Klemmring (15-19) in Umfangsrichtung (L) und/oder Axialrichtung (A) inhomogen ausgelegt ist.

3. Implantat nach Anspruch 1 oder 2, dadurch gekennzeichnet, daß der Klemmring (15-18) Hohlräume (21,27,30-31,35) aufweist.

4. Implantat nach Anspruch 3, dadurch gekennzeichnet, daß die Hohlräume Bohrungen (27,30,30',30'',31) sind.

5. Implantat nach Anspruch 1 oder 2, dadurch gekennzeichnet, daß der Klemmring (19) massiv mit in Umfangsrichtung (L) und/oder Axialrichtung (A) unterschiedlichen Steifigkeitsbereichen (36,19;37,38) ausgelegt ist.

6. Implantat nach einem der vorhergehenden Ansprüche, dadurch gekennzeichnet, daß mindestens eines der Komponenten (12, 41) des Implantats mit einer annähernd ringförmigen Erhebung (28, 46) ausgestattet ist, die im zusammengesetzten Zustand der beiden Komponenten (11,12;40,41) den Klemmringen (17 bzw.15) verformt derart, daß der Klemmring Radialkräfte auf die beiden Komponenten ausübt.

7. Implantat nach einem der vorhergehenden Ansprüche, dadurch gekennzeichnet, daß das Implantat eine zwei- oder mehrteilige Pfanne (10) eines Gelenkimplantats ist.
